# EUROPEAN PATENT APPLICATION

(11) **EP 4 623 932 A1**
(43) Date of publication of application: **01.10.2025**
(21) Application number: 23894640.4
(22) Date of filing: 22.11.2023
(51) Int. Cl.: A61K 45/00, A61K 31/4748, A61K 31/485, A61P 1/02

(54) **AGENT FOR ALLEVIATING CLOZAPINE-INDUCED SIALORRHEA**

(30) Priority: 24.11.2022 JP 2022187772
(71) Applicant: National University Corporation Chiba University, Chiba-shi, Chiba 263-8522 (JP)
(72) Inventor: NIITSU Tomihisa, Chiba-shi, Chiba 260-8670 (JP); ODA Yasunori, Chiba-shi, Chiba 260-8677 (JP); HIROSE Yuki, Chiba-shi, Chiba 260-8675 (JP)
(74) Representative: Hoffmann Eitle
(86) International application number: PCT/JP2023/042038
(87) International publication number: WO 2024/111636

(57) **Abstract**

An object of the present invention to provide a clozapine-induced sialorrhea alleviator. The clozapine-induced sialorrhea alleviator of the present invention contains as an active ingredient dextromethorphan, a compound having an antagonistic action on an NMDA-type glutamate receptor, or a compound having an agonistic action on a sigma-1 receptor.

## Description

### Technical Field

The present invention relates to a clozapine-induced sialorrhea alleviator.

### Background Art

Clozapine, the only approved antipsychotic drug for the treatment of treatment-resistant schizophrenia, is known to cause "clozapine-induced sialorrhea (CIS)" as a side effect, and the deterioration of the patient's quality of life (QOL) is a problem. Currently, an anticholinergic agent having an antagonistic action on a muscarinic receptor is used as a treatment for the sialorrhea.

For the purpose of providing a method for using a muscarinic agonist and an agent using such a muscarinic agonist which may enable a therapeutic effect related to activation of a muscarinic receptor with fewer side effects, PTL1 discloses a method of treating a central nervous system disease, including the step of administering a muscarinic activator to a patient together with a muscarinic inhibitor, wherein the central nervous system disease is selected from schizophrenia, a disorder related to schizophrenia, a muscarinic disorder, a movement disorder, a mood disorder, a cognitive disorder, an attention disorder, and an addictive disorder.

In addition, treatments using anticholinergic drugs (pirenzepine, trihexyphenidyl, biperiden) and α₂-stimulating drugs (clonidine) have been currently reported for CIS (see, NPL1).

### Citation List

### Patent Literature

PTL1: JP 2015-83612 A

### Non Patent Literature

NPL1: David Taylor et al. (Author), Masaru Mimura, Hiroyuki Uchida, Takefumi Suzuki (Translator) "The Maudsley Prescribing Guidelines 14th Edition, Japanese Version", published on October 5, 2022, Wiley Publishing Japan KK

### Summary of Invention

### Technical Problem

The effects of the anticholinergic drugs currently used on alleviation of clozapine-induced sialorrhea are limited, and there is concern that the anticholinergic drugs may attenuate the antipsychotic effects of clozapine and its active metabolite, N-desmethylclozapine, may cause side effects such as memory impairment and constipation (see NPL 1).

An object of the present invention to provide a clozapine-induced sialorrhea alleviator.

### Solution to Problem

The present inventors have found, as a result of intensive studies, that dextromethorphan is effective in alleviating clozapine-induced sialorrhea. The present inventors have also found, as a result of further studies, that a compound having an antagonistic action on an NMDA-type glutamate receptor and a compound having an agonistic action on a sigma-1 receptor is effective in alleviating clozapine-induced sialorrhea, and have completed the present invention.

The present invention includes the following aspects.
<1> A clozapine-induced sialorrhea alleviator containing as an active ingredient a compound having an antagonistic action on an NMDA-type glutamate receptor.
<2> The clozapine-induced sialorrhea alleviator according to <1>, wherein the compound having an antagonistic action on an NMDA-type glutamate receptor is at least one selected from the group consisting of minocycline, WIN 55,212-2, CP 55,940, CPP, aripiprazole, dizocilpine (MK-801), 3-methoxyphencyclidine (3-MeO-PCP), phencyclidine, methoxetamine, ketamine, esketamine, arketamine, norketamine, esnorketamine, arnorketamine, memantine, amantadine, acamprosate, methadone, levomethadone, esmethadone, latrepirdine, tiletamine, selfotel, aptiganel, besonprodil, delucemine, dizocilpine maleate, gabestinel, licostinel, neramexane mesylate, perzinfotel, remacemide hydrochloride, traxoprodil mesylate, D-(-)-2-amino-5-phosphonopentanoic acid (D-AP5), haloperidol, atomoxetine, oseltamivir, kynurenic acid, procaine, felbamate, mephenesin, huperzine A, linalool, orphenadrine, ifenprodil, pentamidine, dextrorphan, and dextromethorphan or a pharmaceutically acceptable salt thereof, or a hydrate thereof.
<3> A clozapine-induced sialorrhea alleviator containing as an active ingredient a compound having an agonistic action on a sigma-1 receptor.
<4> The clozapine-induced sialorrhea alleviator according to <3>, wherein the compound having an agonistic action on a sigma-1 receptor is at least one selected from the group consisting of fluvoxamine, sertraline, S(+)-fluoxetine, (±)fluoxetine, citalopram, imipramine, paroxetine, desipramine, R(-)-fluoxetine, (±)-norfluoxetine, donepezil, dextromethorphan, ifenprodil, SA4503, Dehydroepiandrosterone 3-sulfate (also known as DHEA-S), (+)-SKF10,047, (-)-SKF10,047, (+)-Pentazocine, (-)-Pentazocine, PPBP, Igmesine (also known as JO-1784), PRE-084, Eliprodil (also known as SL 82.0715), DTG203, Pridopidine, and AF710B (also known as aka ANAVEX3-71) or a pharmaceutically acceptable salt thereof, or a hydrate thereof.
<5> A clozapine-induced sialorrhea alleviator containing as an active ingredient dextromethorphan or a pharmaceutically acceptable salt thereof, or a hydration thereof.
<6> The clozapine-induced sialorrhea alleviator according to <5>, wherein a dose of the dextromethorphan or the pharmaceutically acceptable salt thereof, or the hydrate thereof is 0.2 mg/kg/day or more and 3.0 mg/kg/day or less.
<7> A method for alleviating clozapine-induced sialorrhea, including administering an effective amount of a compound having an antagonistic action on an NMDA-type glutamate receptor or a pharmaceutically acceptable salt thereof, or a hydrate thereof.
<8> A method for alleviating clozapine-induced sialorrhea, including administering an effective amount of a compound having an agonistic action on a sigma-1 receptor or a pharmaceutically acceptable salt thereof, or a hydrate thereof.
<9> A method for alleviating clozapine-induced sialorrhea, including administering dextromethorphan or a pharmaceutically acceptable salt thereof, or a hydrate thereof.
<10> Use of a compound having an antagonistic action on an NMDA-type glutamate receptor or a pharmaceutically acceptable salt thereof, or a hydrate thereof, for producing a clozapine-induced sialorrhea alleviator.
<11> Use of a compound having an agonistic action on a sigma-1 receptor or a pharmaceutically acceptable salt thereof, or a hydrate thereof, for producing a clozapine-induced sialorrhea alleviator.
<12> Use of dextromethorphan or a pharmaceutically acceptable salt thereof, or a hydrate thereof, for producing a clozapine-induced sialorrhea alleviator.

### Advantageous Effects of Invention

According to the present invention, a clozapine-induced sialorrhea alleviator is provided.

### Brief Description of Drawings

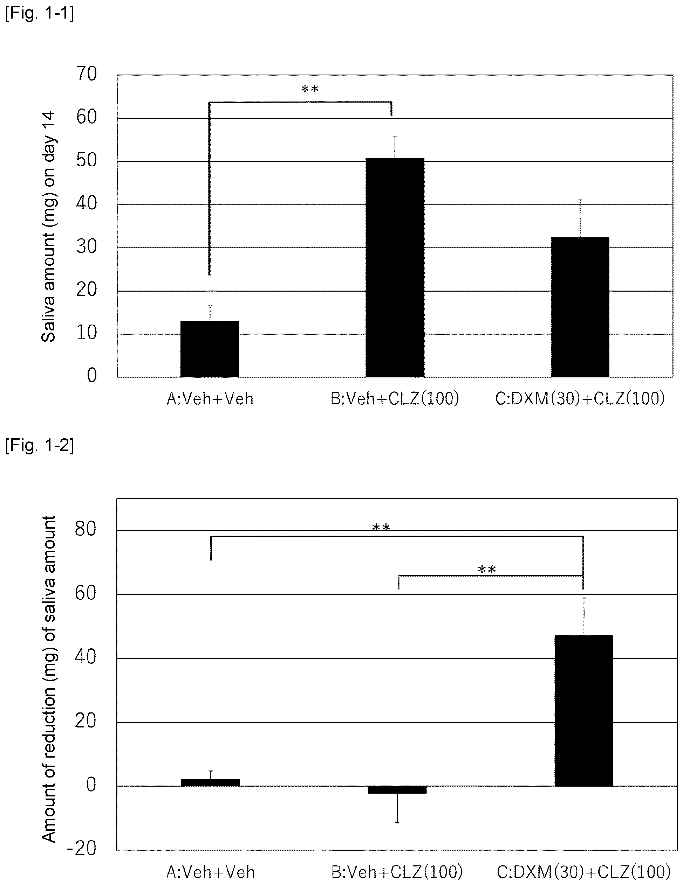

[Fig. 1-1] Fig. 1-1 is a graph showing the results of Experiment 1.
[Fig. 1-2] Fig. 1-2 is a graph showing the results of Experiment 1.
[Fig. 2] Fig. 2 is a graph showing the results of Experiment 2.
[Fig. 3-1] Fig. 3-1 is a graph showing the results of Experiment 3.
[Fig. 3-2] Fig. 3-2 is a graph showing the results of Experiment 3.
[Fig. 4] Fig. 4 is a graph showing the results of Experiment 4.
[Fig. 5] Fig. 5 is a graph showing the results of Experiment 5.

### Description of Embodiments

Hereinafter the present invention will be described in detail with reference to exemplary embodiments, but the present invention is not limited to the embodiments described below.

It should be noted that, unless otherwise stated, all technical and scientific terms used herein have the same meaning as commonly understood by a person skilled in the art to which the present invention belongs. In addition, any materials and methods equivalent or similar to those described herein can be used in the practice of the present invention in a similar manner. All publications and patents cited herein with relation to the invention described herein constitute part of the present specification, for example, as an indication showing methods, materials, and the like that can be used in the present invention.

As used herein, the description "A-B" indicating a numerical range means a numerical range including A and B that are endpoints. The same applies to "A to B".

### [Clozapine-induced sialorrhea alleviator]

A first embodiment of the clozapine-induced sialorrhea alleviator of the present invention contains as an active ingredient dextromethorphan or a pharmaceutically acceptable salt thereof, or a hydrate thereof.

A second embodiment of the clozapine-induced sialorrhea alleviator of the present invention contains as an active ingredient a compound having an antagonistic action on an NMDA-type glutamate receptor.

The reason why a compound having an antagonistic action on an NMDA-type glutamate receptor is effective in alleviating clozapine-induced sialorrhea is considered, but not bound by the following theory, that the activation of an NMDA-type glutamate receptor by clozapine may cause sialorrhea, and the administration of a compound having an antagonistic action on the NMDA-type glutamate receptor alleviates sialorrhea.

A third embodiment of the clozapine-induced sialorrhea alleviator of the present invention contains as an active ingredient a compound having an agonistic action on a sigma-1 receptor.

The reason why a compound having an agonistic action on a sigma-1 receptor is effective in alleviating clozapine-induced sialorrhea is considered, but not bound by the following theory, that the inactivation of a sigma-1 receptor by clozapine may cause sialorrhea, and the administration of a compound having an agonistic action on the sigma-1 receptor alleviates sialorrhea.

In the present invention, a subject to which clozapine-induced sialorrhea alleviator is administered may be any subject with clozapine-induced sialorrhea, preferably any mammal with clozapine-induced sialorrhea. Examples of the subject include human; primates other than human, including non-human primates such as chimpanzees, other apes, and monkey species; domestic animals such as cows, sheep, pigs, goats, and horses; domestic mammals such as dogs and cats; and small animals or laboratory animals including rodents such as mice, rats, and guinea pigs. Among these, it is preferable that the subject is human.

### <Clozapine-induced sialorrhea>

Clozapine-induced sialorrhea is a hypersalivation that is observed as a side effect due to administration of clozapine.

That is, the subject of administration of the clozapine-induced sialorrhea alleviator of the present invention is a patient with treatment-resistant schizophrenia who is receiving treatment with clozapine, and who is also experiencing clozapine-induced sialorrhea due to administration of clozapine.

Clozapine has the following structure:

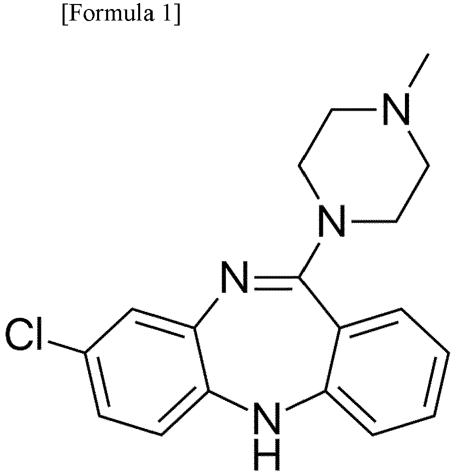

Clozapine is a therapeutic agent for treatment-resistant schizophrenia, and is administered to patients with schizophrenia that is resistant to other antipsychotic treatments (patients who meet the criteria for poor response or intolerance).

In the treatment of treatment-resistant schizophrenia, clozapine is usually administered orally for an adult in the amount of 12.5 mg and 25 mg on the first day and the second day, respectively, once per day. After day 3, the dose is increased by 25 mg a day depending on the symptoms, and the dose is increased to 200 mg per day over 3 weeks in principle. However, when the daily dose exceeds 50 mg, the dose is orally administered in 2 to 3 divided doses. The maintenance dose of clozapine is 200-400 mg per day which is orally administered in 2-3 divided doses, with an appropriate increase or decrease depending on the symptoms. However, each increase in dose should have an interval of 4 days or more and should not exceed 100 mg per day, and the maximum dose should be up to 600 mg per day.

Clozapine-induced sialorrhea is a frequent side effect observed in 46.8% of those taking the drug, according to the package insert of Clozaril (an internal medicine containing clozapine as an active ingredient, manufactured by Novartis Pharmaceuticals Corporation).

### <Compound having antagonistic action on NMDA-type glutamate receptor>

The NMDA-type glutamate receptor (hereinafter also referred to as the "NMDA receptor") is a type of glutamate receptor, and unlike other subtypes of glutamate receptors such as an AMPA receptor and a kainate receptor, N-methyl-D-aspartic acid (NMDA) selectively acts as an agonist.

The compound having an antagonistic action on an NMDA receptor (hereinafter also referred to as an "NMDA receptor antagonist") is not particularly limited, and may be a competitive inhibitor (selective antagonist), may be a functional antagonist such as an open channel inhibitor, and may be an agonist.

The binding site of the selective antagonist is also not particularly limited, and examples thereof include a PCP binding site.

For the NMDA receptor antagonist, see, for example, Reference 1 (Toshiya Inada, Hiroshi Endo, "Compounds that Act on the Excitatory Amino Acid Nervous System," Clinical Psychopharmacology 23: 827-839, 2020, Seiwa Shoten Co., Ltd.). The NMDA receptor antagonist is preferably at least one selected from the group consisting of minocycline, WIN 55,212-2, CP 55,940, 3-[(3R)-3-carboxypiperazin-1-yl]propylphosphonic acid (CPP), aripiprazole, dizocilpine (MK-801), 3-methoxyphencyclidine (3-MeO-PCP), phencyclidine, methoxetamine, ketamine, esketamine, arketamine, norketamine, esnorketamine, arnorketamine, memantine, amantadine, acamprosate, methadone, levomethadone, esmethadone, latrepirdine, tiletamine, selfotel, aptiganel, besonprodil, delucemine, dizocilpine maleate, gabestinel, licostinel, neramexane mesylate, perzinfotel, remacemide hydrochloride, traxoprodil mesylate, D-(-)-2-amino-5-phosphonopentanoic acid (D-AP5), haloperidol, atomoxetine, oseltamivir, kynurenic acid, procaine, felbamate, mephenesin, huperzine A, linalool, orphenadrine, ifenprodil, pentamidine, dextrorphan, and dextromethorphan or a pharmaceutically acceptable salt thereof, or a hydrate thereof.

### <Compound having agonistic action on sigma-1 receptor>

The sigma-1 receptor is a chaperone protein located in the endoplasmic reticulum (ER) and regulates calcium signaling via an IP3 receptor. The sigma-1 receptor is a transmembrane protein expressed in many different tissue types, and it has been reported that it is particularly expressed in certain regions of the central nervous system in large amounts. Although the sigma-1 receptor is considered to be involved in diseases such as schizophrenia, depression, and dementia, its endogenous ligands have not been fully identified.

For the compound having an agonistic action on a sigma-1 receptor (hereinafter also referred to as "sigma-1 receptor agonist"), see, for example, Reference 2 (Narita N, Hashimoto K, Tomitaka S, Minabe Y. Interactions of selective serotonin reuptake inhibitors with subtypes of sigma receptors in rat brain. Eur J Pharmacol. 1996 Jun 20;307(1):117-9. PMID: 8831113 DOI: 10.1016/0014-2999(96)00254-3), Reference 3 (Hashimoto K, Ishiwata K. Sigma receptor ligands: possible application as therapeutic drugs and as radiopharmaceuticals. Curr Pharm Des. 2006; 12(30):3857-76. PMID: 17073684 DOI: 10.2174/138161206778559614), Reference 4 (Nishimura T, Ishima T, Iyo M, Hashimoto K. Potentiation of nerve growth factor-induced neurite outgrowth by fluvoxamine: role of sigma-1 receptors, IP3 receptors and cellular signaling pathways. PLoS One. 2008 Jul 2;3(7):e2558. PMID: 18596927 DOI: 10. 1371/journal.pone.0002558), and Reference 5 (Bogar F, Fulop L, Penke B. Novel Therapeutic Target for Prevention of Neurodegenerative Diseases: Modulation of Neuroinflammation with Sig-1R Ligands. Biomolecules. 2022 Feb 25;12(3):363. PMID: 35327555 DOI: 10.3390/biom12030363). The sigma-1 receptor agonist is preferably at least one selected from the group consisting of fluvoxamine, sertraline, S(+)-fluoxetine, (±)fluoxetine, citalopram, imipramine, paroxetine, desipramine, R(-)-fluoxetine, and (±)-norfluoxetine, which are antidepressants; donepezil that is an antidementia drug, dextromethorphan that is a cough suppressant; experimental reagent SA4503 that is a selective sigma-1 receptor agonist; dehydroepiandrosterone 3-sulfate (also known as DHEA-S) that is an endogenous substances; Ifenprodil, (+)-SKF10,047, (-)-SKF10,047, (+)-Pentazocine, (-)-Pentazocine, PPBP, Igmesine (also known as JO-1784), PRE-084, Eliprodil (also known as SL 82.0715), DTG203, Pridopidine, and AF710B (also known as aka ANAVEX3-71) or a pharmaceutically acceptable salt thereof, or a hydrate thereof.

Dextromethorphan is highly safe to the human body, has a low possibility of side effects caused by combination with clozapine, has a low possibility of attenuating the antipsychotic effect of clozapine, and furthermore has a higher alleviating effect on clozapine-induced sialorrhea. Accordingly, the active ingredient of the clozapine-induced sialorrhea alleviator of the present invention is more preferably at least one selected from the group consisting of dextromethorphan and its active metabolite dextrorphan or a pharmaceutically acceptable salt thereof, or a hydrate thereof, more preferably dextromethorphan or a pharmaceutically acceptable salt thereof, or a hydrate thereof.

Dextromethorphan has the following structure:

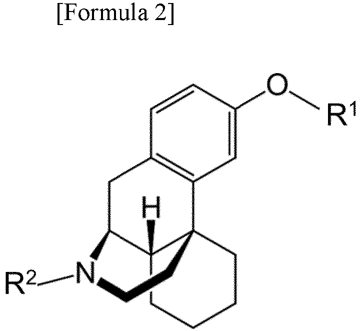

In the formula, R¹ is -CH₃, -CH₂D, -CHD₂, or -CD₃, and R² is -CH₃, -CH₂D, -CHD₂, or -CD₃. Note that D means deuterium.

Dextromethorphan is commercially available under the name of Medicon or the like, and is used as a cough suppressant or the like. Dextromethorphan and a pharmaceutically acceptable salt thereof are also included in generic medicines as a component of an analgesic drug or cough suppressant, and are also used as a pain reliever or opioid enhancer. Dextromethorphan is often used as a hydrobromide hydrate, but dextromethorphan herein is not limited thereto.

Dextromethorphan may be partially deuterated.

Examples of the pharmaceutically acceptable salts include mineral acid salts such as a hydrochloride, a hydrobromide, a hydroiodide, a phosphate, a sulfate, and a nitrate; sulfonates such as a methanesulfonate, an ethanesulfonate, a benzenesulfonate, a p-toluenesulfonate, and a trifluoromethanesulfonate; acid addition salts, for example, organic acid salts such as an oxalate, a tartrate, a citrate, a maleate, a succinate, an acetate, a trifluoroacetate, a benzoate, a mandelate, an ascorbate, a lactate, a gluconate, and a malate; amino acid salts such as a glycine salt, a lysine salt, an arginine salt, an ornithine salt, a glutamate salt, and an aspartate salt; or base addition salts, for example, inorganic salts such as a lithium salt, a sodium salt, a potassium salt, a calcium salt, and a magnesium salt, or salts with an organic base such as an ammonium salt, a triethylamine salt, a diisopropylamine salt, a cyclohexylamine salt, and an N-methyl-D-glucamine salt. The salt includes an aqueous salt.

In the present invention, the exemplified NMDA receptor antagonist and sigma-1 receptor agonist may have an asymmetric center, and in this case, various optical isomers are present. The NMDA receptor antagonist and the sigma-1 receptor agonist may be present as separate optically active compounds of (R) and (S), and as a racemate or (RS) mixture, as long as they have an antagonistic action on the NMDA receptor and an agonistic action on the sigma-1 receptor, respectively. In the case of compounds having two or more asymmetric centers, there are also diastereomers due to each optical isomerism, and the NMDA receptor antagonist and the sigma-1 receptor agonist may be a mixture including all of these forms in any ratio. For example, a diastereomer can be separated by a method well known to those skilled in the art, such as a fractional crystallization method, and an optically active compound can be obtained by a well-known organic chemical method for this purpose.

In addition, the exemplified NMDA receptor antagonist and sigma-1 receptor agonist may be present as a geometric isomer such as a cis-compound or a trans-compound. Furthermore, the NMDA receptor antagonist and sigma-1 receptor agonist may have tautomerism and a variety of tautomers may be present. The compound of the present invention may be one of these isomers or a mixture containing these isomers at any ratio.

Furthermore, when the exemplified NMDA receptor antagonist and sigma-1 receptor agonist or a salt thereof form a hydrate or solvate, the hydrate and solvate are also included within the scope of the present invention.

When the active ingredient of the clozapine-induced sialorrhea alleviator of the present invention is dextromethorphan or a pharmaceutically acceptable salt thereof, or a hydrate thereof (hereinafter also referred to as dextromethorphan and the like), the dose of dextromethorphan and the like to a human is preferably 0.2 mg/kg/day or more and 3.0 mg/kg/day or less, more preferably 0.5 mg/kg/day or more, and more preferably 1.0 mg/kg/day or more.

The number of doses per day of dextromethorphan and the like to a human is not particularly limited, but is preferably 1 or more and 5 or less times, more preferably 2 or more times, more preferably 3 or more, and it is preferably 4 or less times.

The daily dose of dextromethorphan and the like to a human is preferably 10 mg/day or more and 120 mg/day or less, more preferably 15 mg/day or more, more preferably 30 mg/day or more, still more preferably 45 mg/day or more.

For the clozapine-induced sialorrhea alleviator of the present invention, dextromethorphan, the NMDA receptor antagonist, or the sigma-1 receptor agonist contained therein can be administered alone or with a pharmacologically or pharmaceutically acceptable additive.

As the additive, excipient or diluent for regular use, and, if necessary, a commonly-used binder, disintegrant, lubricant, coating agent, sugar coating agent, pH adjusting agent, solvilizing agent, or aqueous or non-aqueous solvent can be used. Specific examples thereof include water, lactose, dextrose, fructose, sucrose, sorbitol, mannitol, polyethylene glycol, propylene glycol, potato starch, corn starch, gum, gelatin, alginate, calcium silicate, calcium phosphate, cellulose, liquid sugar, methyl cellulose, polyvinylpyrrolidone, alkyl parahydroxybenzoate, talc, stearic acid, magnesium stearate, agar, pectin, gum arabic, glycerin, sesame oil, olive oil, soybean oil cocoa butter, ethylene glycol, low viscosity hydroxypropyl cellulose (HPC-L), microcrystalline cellulose, carboxymethyl cellulose (CMC), sodium carboxymethyl cellulose (CMC-Na) or the like, and other additives for regular use. A hydrate or the like of the compound described above may also be used.

As the clozapine-induced sialorrhea alleviator of the present invention, a commercially available formulation or future commercially available formulation or combination containing dextromethorphan, an NMDA receptor antagonist, or a sigma-1 receptor agonist may be used. Examples thereof include Medicon, a cough suppressant, or the like, and AVP-786, a combination of deuterated dextromethorphan and quinidine developed by Otsuka Pharmaceutical Co., Ltd., or the like.

The clozapine-induced sialorrhea alleviator according to the present invention may be in any form of a solid composition, a liquid composition, and other compositions, and an optimal form is selected as necessary.

The clozapine-induced sialorrhea alleviator of the present invention can be prepared into a tablet, a pill, a capsule, a granule, a powder, particles, a liquid, an emulsion, a suspension, an injection, a patch, and the like by adding the aforementioned additives to dextromethorphan, an NMDA receptor antagonist, or a sigma-1 receptor agonist, using conventional formulation techniques.

In addition, the clozapine-induced sialorrhea alleviator of the present invention can be formulated by forming an inclusion compound of dextromethorphan, an NMDA receptor antagonist, or a sigma-1 receptor agonist with α, β, or γ-cyclodextrin, methylated cyclodextrin, or the like.

The clozapine-induced sialorrhea alleviator of the present invention can be prepared into a single formulation (combination drug) or separately-formulated two or more formulations (concomitant drug) using a compound that can be used in combination with dextromethorphan, an NMDA receptor antagonist, or a sigma-1 receptor agonist. Examples of the compound that can be used in combination include clozapine and alleviators of other side effects of clozapine, stabilizers of NMDA receptor antagonists, and the like.

When these compounds are formulated separately into two or more formulations, the individual formulations can be administered simultaneously or with certain time intervals. In this case, the order in which they are administered may not matter. The two or more formulations each can be administered at different number of doses per day. The two or more formulations can also be administered by different routes.

When these compounds are formulated separately into two or more formulations, the formulations may be administered simultaneously or with very short intervals, and it is preferable to indicate that they are used in combination in a document such as a package insert or sales brochure of a commercially available drug.

It is also preferred that these active ingredients are formulated separately into the form of a kit consisting of two formulations.

Dextromethorphan, the NMDA receptor antagonist, or the sigma-1 receptor agonist may be administered orally as is. Dextromethorphan, the NMDA receptor antagonist, or the sigma-1 receptor agonist may also be administered orally as an agent including dextromethorphan, the NMDA receptor antagonist, or the sigma-1 receptor agonist as an active ingredient.

The dose and administration schedule of dextromethorphan, the NMDA receptor antagonist, or the sigma-1 receptor agonist vary depending on the subject of administration, the route of administration, the symptoms, and the like. For example, when dextromethorphan, the NMDA receptor antagonist, or the sigma-1 receptor agonist is administered orally to an adult patient, one dose is usually 0.1 mg to 1000 mg, preferably 1 mg to 200 mg, and this amount is preferably administered once to five times per day or once per two to three days.

### Examples

The present invention will be described more specifically by way of the following Examples, but the Examples are merely illustrative of the present invention and do not limit the scope of the present invention.

### [Creation of CIS model animals]

CIS model animals were created with reference to previous studies (Ishikawa S, J. Pharmacol. Exp. Ther., 2020, Nov; 375(2): 376-384).

As experimental animals, 6 to 8-week-old male Wister rats (made by CLEA Japan, Inc.) were used. The animals were freely ingested and fed on water and normal feed except for the period of time from 1 hour before the saliva amount measurement described below to the measurement. The agent to be administered, clozapine, was used by dissolving a 100 mg tablet of Clozaril (manufactured by Novartis Pharmaceuticals Corporation) in distilled water.

A single oral dose (1 mL/kg) of 25 mg/kg/day, 50 mg/kg/day, or 100 mg/kg/day clozapine was administered to the rats, and the amount of saliva was measured 4 times per day, every 6 hours from 1 hour after administration, but no increase in the amount of saliva secretion was observed.

25 mg/kg/day, 50 mg/kg/day, or 100 mg/kg/day of clozapine was also administered orally to the rats once per day (1 mL/kg), and the saliva amount was measured after 7 days of administration and 24 hours after the final administration. As a result, the saliva amount did not increase in the 25 mg/kg/day administration group, but increased significantly in the 50 mg/kg/day administration group and the 100 mg/kg/day administration group from day 3 of administration.

From the above, rats to which 100 mg/kg/day clozapine (1 mL/kg) was administered orally once per day for 7 consecutive days were adopted as CIS model animals.

The method for measuring the saliva amount was as follows.

### <Saliva Amount Measurement>

Water and feed were limited from 1 hour before the saliva amount measurement.

As a method for measuring the amount of saliva, a small piece of cotton ball was placed in the mouth of the restrained rat and swabbed for 10 seconds (the saliva was absorbed into the cotton ball). This was repeated three times, and the mass change of the cotton ball was measured. The mass change was measured as the saliva amount collected over a total of 30 seconds.

### [Experiment 1]

The dose and period of administration of the NMDA receptor antagonist dextromethorphan (DXM) were investigated using the above-described CIS model animals (CIS model rats). Experiment 1 was performed on CIS model rats of groups A to C as follows.

### <Day 1 to Day 7>

Group A (control): Oral administration of distilled water (DW) (1 mL/kg) once per day
Groups B and C: Oral administration of clozapine 100 mg/kg/day (1 mL/kg) once per day

### <Day 8 to Day 14>

Group A: Intraperitoneal administration of saline (1 mL/kg) + oral administration of distilled water (DW) (1 mL/kg) once per day
Group B: Intraperitoneal administration of saline (1 mL/kg) + oral administration of clozapine 100 mg/kg/day (1 mL/kg) once per day
Group C: Intraperitoneal administration of DXM (30 mg/kg/day) + oral administration of clozapine 100 mg/kg/day (1 mL/kg) once per day

### <Day 14>

The saliva amount was measured before drug administration on day 14 (24 hours after drug administration up to day 13).

### <Results>

The saliva amount on day 14 was significantly increased in group B, where clozapine was administered, compared to group A (control group) (p < 0.01). In group C, where dextromethorphan in addition to clozapine were administered on days 8 to 13, reduction of saliva amount was observed on day 14. The results are shown in Fig. 1-1.

When the change in saliva amount on day 14 was compared based on the saliva amount on day 7, it was significantly reduced in group C (DXM 30 mg/kg/day) compared to groups A and B (p < 0.01). The results are shown in Fig. 1-2.

### [Experiment 2]

In the above Experiment 1, the saliva amount was measured 24 hours after DXM administration. Since DXM has a short half-life in humans (3.2 ± 0.3 to 3.6 ± 0.3 hr), the acute effect was investigated by measuring the saliva amount 1 hour after administration.

The following studies were performed on the acute phase effect of dextromethorphan (DXM) using the above-described CIS model animals (CIS model rats).

### <Day 1 to Day 7>

Groups A and B: Clozapine at 100 mg/kg/day (1 mL/kg) was orally administered once per day. Swabs were conducted on a daily basis to accustom the model animals with saliva measurements.

### <Day 8 to Day 9>

Group A: Intraperitoneal administration of saline (1 mL/kg) once per day
Group B: Intraperitoneal administration of DXM (30 mg/kg/day) once per day

On Day 9, the saliva amount was measured 1 hour after DXM administration.

### <Results>

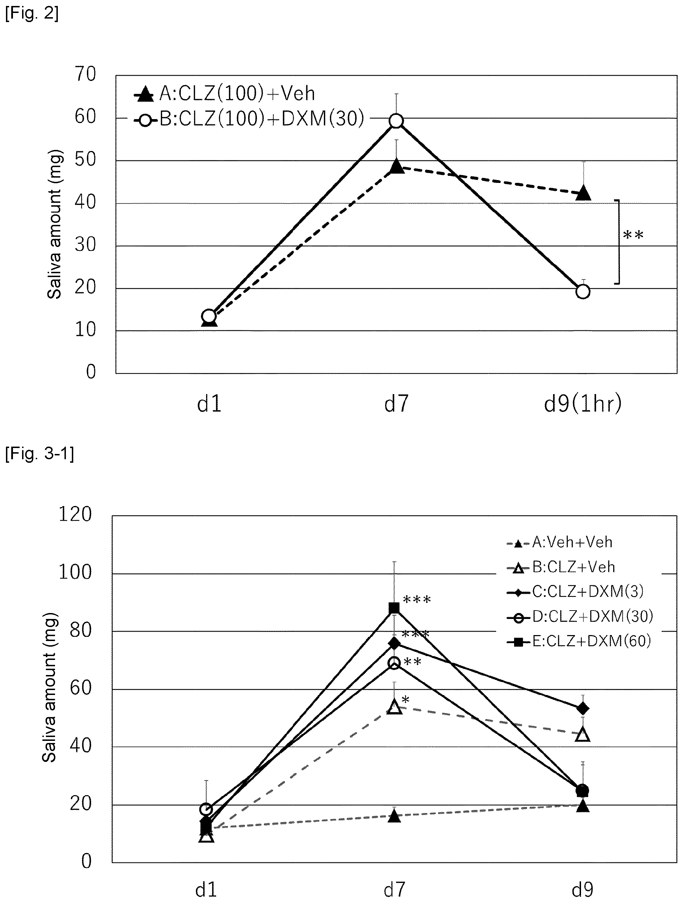

On day 7 (d7), the saliva amount was significantly increased compared to day 1 (d1) (p > 0.01) due to administration of clozapine. In group B, where dextromethorphan (DXM 30 mg/kg/day) in addition to clozapine were administered on days 8 and 9, the saliva amount was significantly reduced (p < 0.01) at 1 hour after administration on day 9 (d9). The results are shown in Fig. 2.

### [Experiment 3]

The dose of dextromethorphan (DXM) was changed to examine the dose-dependency of clozapine-induced sialorrhea suppression effect.

The following studies were performed on the dose-dependency of the acute phase effect of DXM using the above-described CIS model animals (CIS model rats).

### <Day 1 to Day 7>

Group A (control): Oral administration of distilled water (DW) (1 mL/kg) once per day
Groups B, C, D and E: Oral administration of clozapine 100 mg/kg/day (1 mL/kg) once per day
Note that swabs were performed on days 5 and 6 to accustom the model animals with the saliva measurement.

### <Day 8 to Day 9>

Group A: Intraperitoneal administration of saline (1 mL/kg) for a total of 3 times
Group B: Intraperitoneal administration of saline (1 mL/kg) for a total of 3 times
Group C: Intraperitoneal administration of DXM (3 mg/kg) for a total of 3 times
Group D: Intraperitoneal administration of DXM (30 mg/kg) for a total of 3 times
Group E: Intraperitoneal administration of DXM (60 mg/kg) for a total of 3 times

The agent of the above dose was administered once in the morning and once in the afternoon on day 8 and once in the morning on day 9 for a total of 3 times. The saliva amount was measured 1 hour after the final administration.

### <Results>

The saliva amount on day 7 was significantly increased due to clozapine compared to group A (control) (p < 0.01). The results are shown in Fig. 3-1.

Compared to group A, the significant difference in group B was P < 0.05, the significant difference in group D was P < 0.01, and the significant difference in group C was P < 0.001.

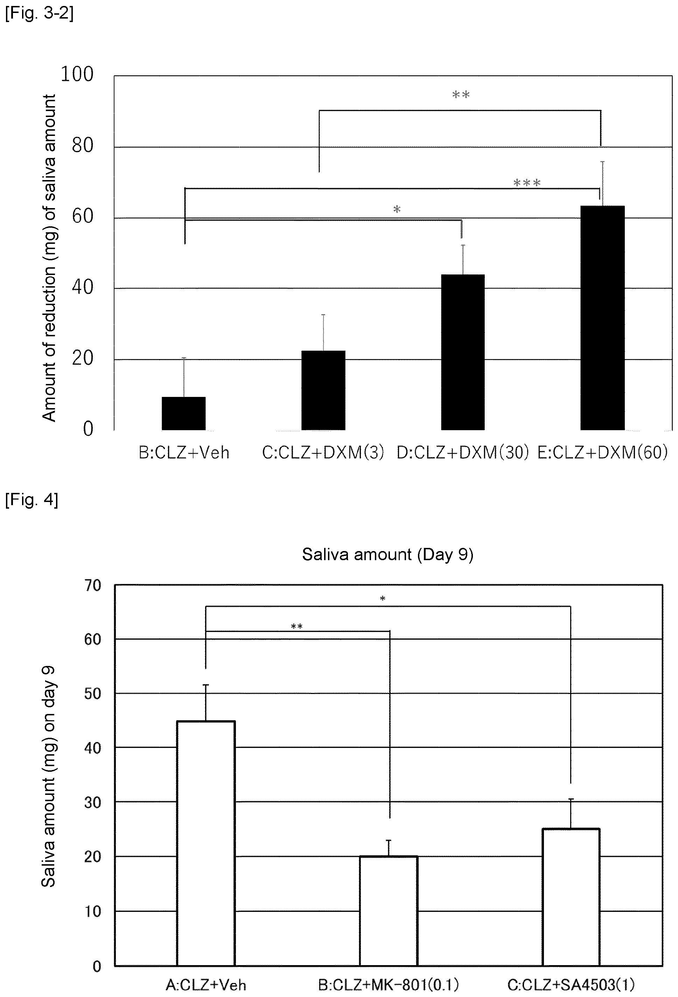

When the change in saliva amount was compared from day 7 to day 9 (1 hour after administration), the saliva amount was significantly reduced in group D (DXM 30 mg/kg) and group E (DXM 60 mg/kg) (p < 0.05 and p < 0.001). In addition, the saliva amount was significantly reduced in group E (DXM 60 mg/kg) compared to group C (DXM 3 mg/kg) (p < 0.01). The results are shown in Fig. 3-2.

The results of Experiments 1 and 2 showed that the administration of dextromethorphan significantly reduced the amount of saliva in the model rats with clozapine-induced sialorrhea. Furthermore, the results of Experiment 3 showed that the effect of dextromethorphan was dose-dependent.

### [Experiment 4]

The alleviating effect (acute phase effect) on clozapine-induced sialorrhea by a selective NMDA receptor antagonist MK-801 and a selective sigma-1 receptor agonist SA4503 were investigated. Experiment 4 was performed on CIS model rats of groups A to C as follows.

### <Day 1 to Day 7>

Groups A (control), B, and C: Oral administration of clozapine 100 mg/kg/day (1 mL/kg) once per day

### <Day 8 to Day 9>

Group A (control): Intraperitoneal administration of saline (1 mL/kg) for a total of 3 times
Group B: Intraperitoneal administration of MK-801 (0.1 mg/kg) for a total of 3 times
Group C: Intraperitoneal administration of SA4503 (1 mg/kg) for a total of 3 times

Note that the intraperitoneal administration was performed once in the morning and once in the afternoon on day 8 and once in the morning on day 9 for a total of 3 times. The saliva amount was measured 1 hour after the final administration.

### <Results>

The results of Experiment 4 are shown in Fig. 4.

In the administration group of a selective NMDA receptor antagonist MK-801 (group B), the saliva amount was significantly reduced on day 9 compared to group A (control) (p < 0.01).

Similarly, in the administration group of a selective sigma-1 receptor agonist SA4503 (group C), the saliva amount was significantly reduced on day 9 compared to group A (control group) (p < 0.05).

The results of Experiment 4 showed that the administration of an NMDA receptor antagonist and a sigma-1 receptor agonist significantly reduced the saliva amount in model rats with clozapine-induced sialorrhea.

### [Experiment 5]

Regarding the alleviating effect of the selective NMDA receptor antagonist MK-801 on clozapine-induced sialorrhea, the alleviating effect (acute phase effect) on clozapine-induced sialorrhea when the selective NMDA receptor antagonist MK-801 was used in combination with D-serine or Glycine, which are NMDA receptor agonists, was investigated. Experiment 5 was performed on CIS model rats of groups A to D as follows.

### <Day 1 to Day 7>

Groups A (control), B, C, and D: Oral administration of clozapine 100 mg/kg/day (1 mL/kg) once per day

### <Day 8 to Day 9>

Group A (control): The solvent (5 mL/kg) was orally administered, and then 1 hour later, saline (1 mL/kg) was intraperitoneally administered. This administration was performed for a total of 3 times.

Group B: The solvent (5 mL/kg) was orally administered, and then 1 hour later, MK-801 (0.1 mg/kg) was intraperitoneally administered. This administration was performed for a total of 3 times.

Group C: D-serine (900 mg/kg) was orally administered, and then 1 hour later, MK-801 (0.1 mg/kg) was intraperitoneally administered. This administration was performed for a total of 3 times.

Group D: Glycine (900 mg/kg) was orally administered, and then 1 hour later, MK-801 (0.1 mg/kg) was intraperitoneally administered. This administration was performed for a total of 3 times.

Note that the administration was performed once in the morning and once in the afternoon on day 8 and once in the morning on day 9 for a total of 3 times. The saliva amount was measured 1 hour after the final administration.

### <Results>

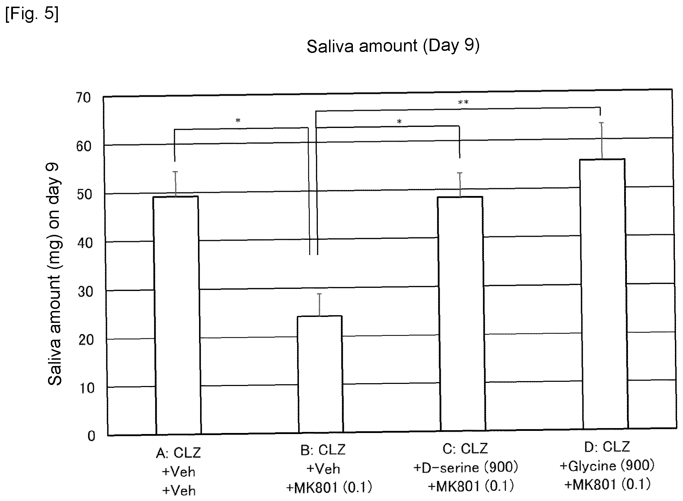

The results of Experiment 5 are shown in Fig. 5.

In the administration group of a selective NMDA receptor antagonist MK-801 (group B), the saliva amount was significantly reduced on day 9 compared to group A (control) (p < 0.05).

In group C, where the NMDA receptor agonist D-serine was administered in addition to MK-801, the reduction in saliva amount was significantly antagonized compared to group B (p < 0.05), resulting in a saliva amount of the same level as that in group A.

Similarly, in group D, where the NMDA receptor agonist glycine was administered in addition to MK-801, the reduction in saliva amount was significantly antagonized compared to group B (p < 0.01), resulting in a saliva amount of the same level as that in group A.

The results of Experiment 5 showed that clozapine-induced sialorrhea is alleviated with an NMDA receptor antagonist, and the alleviating effect is antagonized with an NMDA receptor agonist.

### Industrial Applicability

It was shown that clozapine-induced sialorrhea, which is a side effect of clozapine that is a therapeutic agent for treatment-resistant schizophrenia, is alleviated by dextromethorphan, a compound having an inhibitory effect on an NMDA-type glutamate receptor, or a compound having an agonistic action on a sigma-1 receptor.

Dextromethorphan has already been used as a cough suppressant and the like, and its safety to the human body has been well evaluated, thus a highly safe clozapine-induced sialorrhea alleviator is provided.

## Claims

1. A clozapine-induced sialorrhea alleviator comprising as an active ingredient a compound having an antagonistic action on an NMDA-type glutamate receptor.

2. The clozapine-induced sialorrhea alleviator according to claim 1, wherein the compound having an antagonistic action on an NMDA-type glutamate receptor is at least one selected from the group consisting of minocycline, WIN 55,212-2, CP 55,940, CPP, aripiprazole, dizocilpine (MK-801), 3-methoxyphencyclidine (3-MeO-PCP), phencyclidine, methoxetamine, ketamine, esketamine, arketamine, norketamine, esnorketamine, arnorketamine, memantine, amantadine, acamprosate, methadone, levomethadone, esmethadone, latrepirdine, tiletamine, selfotel, aptiganel, besonprodil, delucemine, dizocilpine maleate, gabestinel, licostinel, neramexane mesylate, perzinfotel, remacemide hydrochloride, traxoprodil mesylate, D-(-)-2-amino-5-phosphonopentanoic acid (D-AP5), haloperidol, atomoxetine, oseltamivir, kynurenic acid, procaine, felbamate, mephenesin, huperzine A, linalool, orphenadrine, ifenprodil, pentamidine, dextrorphan, and dextromethorphan or a pharmaceutically acceptable salt thereof, or a hydrate thereof.

3. A clozapine-induced sialorrhea alleviator comprising as an active ingredient a compound having an agonistic action on a sigma-1 receptor.

4. The clozapine-induced sialorrhea alleviator according to claim 3, wherein the compound having an agonistic action on a sigma-1 receptor is at least one selected from the group consisting of fluvoxamine, sertraline, S(+)-fluoxetine, (±)fluoxetine, citalopram, imipramine, paroxetine, desipramine, R(-)-fluoxetine, (±)-norfluoxetine, donepezil, dextromethorphan, ifenprodil, SA4503, Dehydroepiandrosterone 3-sulfate (also known as DHEA-S), (+)-SKF10,047, (-)-SKF10,047, (+)-Pentazocine, (-)-Pentazocine, PPBP, Igmesine (also known as JO-1784), PRE-084, Eliprodil (also known as SL 82.0715), DTG203, Pridopidine, and AF710B (also known as ANAVEX3-71) or a pharmaceutically acceptable salt thereof, or a hydrate thereof.

5. A clozapine-induced sialorrhea alleviator comprising as an active ingredient dextromethorphan or a pharmaceutically acceptable salt thereof, or a hydration thereof.

6. The clozapine-induced sialorrhea alleviator according to claim 5, wherein a dose of the dextromethorphan or the pharmaceutically acceptable salt thereof, or the hydrate thereof is 0.2 mg/kg/day or more and 3.0 mg/kg/day or less.
